**Europäisches Patentamt**

**European Patent Office**

**Office européen des brevets**

(19)

(11) Veröffentlichungsnummer : **0 065 175 B2**

(12) **NEUE EUROPÄISCHE PATENTSCHRIFT**

(45) Veröffentlichungstag der neuen Patentschrift :
05.06.91 Patentblatt 91/23

(51) Int. Cl.$^5$ : **A61K 7/09**

(21) Anmeldenummer : **82103796.7**

(22) Anmeldetag : **04.05.82**

(54) **Verfahren zur dauerhaften Verformung von Haaren.**

Die Akte enthält technische Angaben, die nach dem Eingang der Anmeldung eingereicht wurden und die nicht in dieser Patentschrift enthalten sind.

(30) Priorität : 16.05.81 DE 3119634

(43) Veröffentlichungstag der Anmeldung :
24.11.82 Patentblatt 82/47

(45) Bekanntmachung des Hinweises auf die Patenterteilung :
27.03.85 Patentblatt 85/13

(45) Bekanntmachung des Hinweises auf die Entscheidung über den Einspruch :
05.06.91 Patentblatt 91/23

(84) Benannte Vertragsstaaten :
**AT BE CH DE FR GB IT LI NL SE**

(56) Entgegenhaltungen :
CH-A- 307 483
DE-A- 1 467 912
DE-A- 2 822 125
DE-B- 1 012 435
DE-B- 1 067 565
DE-B- 1 089 124
DE-B- 1 126 071
DE-B- 1 139 608
DE-B- 2 263 203
FR-A- 1 437 447
GB-A- 1 105 876
GB-A- 1 187 568
GB-A- 1 198 857
GB-A- 1 230 494
US-A- 2 689 815
US-A- 2 738 304
US-A- 3 837 349
US-A- 4 214 596
PATENTS ABSTRACTS OF JAPAN, Band 4, Nr. 93(C-17)(575), 5. Juli 1980, Seite 18C17
"Cosmetics - Science and Technology" Band 3, (1972) Seiten 2/7-8
Friseurwelt, 05.06.1975, S. 43
Ullmanns Enzyklopädie der Technischen Chemie, 3. Auflage, Band 10, 1958, S. 747
Werbedruckschrift "Techneed Products Line" der Firma SUNSTAR

(56) Entgegenhaltungen :
**Japanische Werbedrucksache "New Hair Collection 78"**
**Japanisches Haarlexikon 1978, S. 197-198**
**Schwarzkopf Wellfibel, S. 20-21, 40-41**
**Zeitschrift "Das Haar", Jahrgang 7, Nr. 9/10, 1950, S. 63-64**

(73) Patentinhaber : **Wella Aktiengesellschaft**
**Berliner Allee 65**
**W-6100 Darmstadt (DE)**

(72) Erfinder : **Wajaroff, Theodor**
**Erbacher Strasse 56 b**
**W-6100 Darmstadt (DE)**

EP 0 065 175 B2

EP 0 065 175 B2

## Beschreibung

Es sind bereits Mittel zur Haarbehandlung, u.a. auch zur Haarverformung, bekannt geworden, welche aus einem Sulfit als Verformungswirkstoff, einem Quellstoff für das Haar und einem Verdikkungsstoff zur Bildung einer hohen Viskosität der wäßrigen Lösungen bestehen. Der Verdickunffsstoff, wie insbesondere Guargummi, soll in einer solchen Menffe in dem Mittel enthalten sein, daß eine endgültige scheinbare Viskosität von mindestens 350 cp erreicht wird (vgl. DE-A-1 467 912).

Ferner wurden schon Dauerwellmittel für Kaltwell- oder Heißwellverfahren beschrieben, welche durch Gelatine in Gelform gebracht sind und neben anderen wirksamen Stoffen noch Zucker, vorzuffsweise Disaccharide, enthalten. Die Mittel sollen beim Behandeln des Haares keinen Glitscheffekt verursachen, auf dem Haar einen eng haftenden Film bilden und sich leicht auswaschen lassen (vgl. DE-B-1 012 435).

Ein Verfahren zur Dauerwellung oder Entkräuselung menschlicher Haare, bei dem man die Haare zunächst mit einem wäßrigen, neutralen oder alkalischen Verformungsmittel auf der Basis eines wasserlöslichen Salzes der Thioglykol- oder Thiomilchsäure behandelt und anschließend ein unmittelbar vor der Anwendung hergestelltes weiteres wäßriges Verformungsmittel der ansonsten gleichen Zusammensetzung, jedoch einem zusätzlichen Gehalt an einem hydrophilen Ester der Thioglykol- oder Thiomilchsäure, aufträgt, ist aus der DE-B-2 263 203 bekannt. Der zugesetzte Ester der Thioglykol- bzw. Thiomilchsäure wird allmählich in der alkalischen Lösung verseift, wodurch die zunächst erhöhte Verformungswirksamkeit des Verformungsmittel-Gemisches allmählich abnimmt.

Weiterhin sind bereits alkalische Mittel zur Dauerwellung von Haaren bekannt, die solche Mengen eines Cellulosederivates als Verdicker enthalten, daß sie eine creme- oder gelartige Konsistenz besitzen. Vorzugsweise soll als Cellulosederivat die dermatologisch unschädliche Tylose verwendet werden. Wie erwähnt ist, sind die Mittel handlich im Gebrauch, lassen sich leicht in Tuben abfüllen und genau auf die gewünschten Haarpartien aufbringen (vgl CH-A-307 483).

Außerdem wurden schon undurchsichtiffe Haarverformungsmittel mit einem Gehalt an keratinreduzierenden Wirkstoffen beschrieben, welche in einer feinverteilten wäßrigen Dispersion eines stark gesättifgten amorphen festen Polystyrols und etwa 100-1000°% in bezug auf das Polystyrol eines nichtionischen Dispersionsstoffes gelöst sind. Diese Mittel, die sowohl für die Dauerwellung als auch für die Entkräuselung geeignet sein sollen, besitzen die erwünschte Undurchsichtigkeit und stellen stabile ansehnliche weiße, cremig erscheinende Präparate dar (vgl. US-A-2 689 815).

Es sind weiterhin 2-Komponenten-Kaltwellösungen bekannt, bei denen die eine Komponente eine Flüssigkeit mit einem Gehalt an einem Reduktionsmittel und die andere Komponente eine Flüssigkeit mit einem Gehalt an einem Oxydationsmittel darstellt. Beide Flüssigkeiten enthalten ferner einen gelbildenden Stoff, einen die Gelbildung fördernden Stoff, ein flüchtiges Lösungsmittel wie Ethanol sowie Wasser. Durch das flüchtige Lösungsmittel wird die Gelbildung vor der Anwendung verhindert, während es nach der Anwendung rasch verdunstet und dadurch die Gelbildung der Flüssigkeiten bewirkt. Wie erwähnt ist, läßt sich durch diese Mittel das Abtropfen der Lösung vermeiden und die Einwirkungszeit verringern (vgl. Patents Abstracts of Japan, Band 4, Nr. 93 (C-17) (575) (1980), Seite 18c 17).

Ferner sind auch Mittel sowie ein Verfahren zur Dauerwellung von Haaren offenbart worden, wobei die Mittel wäßrige Pasten darstellen, die eine wellwirksame Substanz wie insbesondere ein Thioglykolat und außerdem einen Verdicker wie vorzugsweise Montmorillonit oder ein Alginat enthalten. Die Viskosität der Mittel soll ausreichend sein, um eine Wellung des Haares in der gewünschten Form zu erhalten. Das Verfahren besteht im wesentlichen darin, daß die Haarwellung allein mit Hilfe des pastösen Mittels aufrechterhalten wird, während die Strähne in Kontakt mit dem wellwirksamen Stoff ist (vgl. FR-A-1 437 447).

Schließlich wurden schon Haarverformungsmittel in Creme- und Gelform auf Grund ihrer Bestandteile und Rezepturen zusammenfassend näher erläutert. Hierbei wird für Haarentkräuselungsmittel eine steife, für Dauerwellmittel eine weiche Zustandsform als zweckmäßiff empfohlen. Allgemein sollen diese Mittel nicht tropfen und dem Haar guten Griff sowie Glanz verleihen (vgl. Balsam and Sagarin, "Cosmetics-Science and Technology" Band 2 (1972), Seiten 217/218).

Bei den üblichen Haarverformungsverfahren werden die Haare zunächst mit der wäßrigen Lösung einer reduzierenden Verbindung, insbesondere einer Mercaptoverbindung, vor allem mit Thioglykolat in alkalischem Medium, oder aber mit Sulfit, vorzugsweise in schwach saurem bis neutralem Medium, behandelt. Hierbei werden die Disulfidbrückenbindungen des Haarkeratins durch chemische Reäktion gespalten und die Haare einer dauerhaften Verformung zugänglich.

Die praktische Durchführung der dauerhaften Verformung von menschlichen Haaren erfolgt im allgemeinen nach zwei Varianten. Nach einer Variante teilt man das gewaschene und handtuchtrockene Haar zunächst in mehrere Partien auf, feuchtet diese Partien sodann mit einem Teil eines flüssigen Dauerverformungsmittels vor und wickelt anschließend auf Wickler. Nach Beendigung des Wickelvorgangs werden die Wickler mit dem

2

Rest des flüssigen Dauerverformungsmittels nachgefeuchtet. Bei der anderen Variante teilt man nach vorangegangener Haarwäsche das handtuchtrocken gemachte Haar ebenfalls zunächst in mehrere Partien auf und wickelt sie anschließend, ohne mit einem Dauerverformungsmittel vorzufeuchten, auf Wickler. Nach Beendigung des Wickelvorganges werden die Wickler mit der gesamten dafür erforderlichen Menge des Dauerverformungsmittels gründlich durchgefeuchtet. Die für eine Dauerwellung verwendeten Wickler haben einen Durchmesser von etwa 5 bis 13 Millimeter, während für eine Haarentkräuselung Wickler mit einem Durchmesser von über 13 Millimeter erforderlich sind.

Die Einwirkungszeit des Haarverformungsmittels auf das Haar beträgt sowohl bei der Dauerwellung als auch bei der dauerhaften Entkräuselung, je nach Haarbeschaffenheit und dem gewünschten Grad der Umformung im allgemeinen bis zu etwa 30 Minuten. Durch Wärmezufuhr, beispielsweise unter Verwendung eines Wärmestrahlers oder einer Trockenhaube, läßt sich die Einwirkungszeit verkürzen.

Nach Ablauf der erforderlichen Einwirkungszeit des Haarverformungsmittels wird das gewickelte Haar mit Wasser gespült und mit der wäßrigen Lösung eines Oxidationsmittels, vorzugsweise mit einer etwa 2%igen Hydrogenperoxidlösung, unter Wiederverknüpfung der zuvor gespaltenen Disulfidbindungen des Haarkeratins in der neuen Form fixiert.

Die Einwirkzeit des Fixiermittels beträgt hierbei üblicherweise etwa 10 bis 15 Minuten. Schließlich werden die Wickler entfernt und das Haar gründlich mit Wasser ausgespült. Während der Einwirkungszeit des Haar-verformungsmittels, und besonders bei Zufuhr von Wärme, entweicht ein großer Teil des als Alkalisierungsmittel in den Präparaten vorhandenen Ammoniaks. Außerdem werden, bedingt durch die große Oberfläche des Haares, Teile der als reduzierend wirkende Wellwirkstoffe enthaltenen Mercaptoverbindung oder des Sulfits durch den Luftsauerstoff oxidiert. Ist das Haarverformungsmittel sauer eingestellt, so kann sogar eine Zersetzung des Wellwirkstoffs unter Abspaltung von übelriechendem Schwefelwasserstoff erfolgen. Die fehlende Abschirmung der Wickler hat schließlich einen Verlust an Flüssigkeit und Wärme zur Folge. Die genannten Effekte treten naturgemäß an den äußeren Lagen des gewickelten Haares, also im Bereich des Haaransatzes, stärker auf als an den inneren Lagen des Haares. Infolgedessen ist die Dauerverformung im Bereich des Haaransatzes unbefriedigend. Verlängert man hingegen die Einwirkungszeit oder wählt ein stärker verformungswirksames Mittel, so erhält man zwar eine ausreichende Verformung im Bereich des Haaransatzes, jedoch werden die Haarspitzen geschädigt.

Dieses negative Ergebnis wird außerdem dadurch verstärkt, daß die Haarspitzen gegenüber chemischen Einwirkungen ohnehin empfindlicher sind als der Haaransatz. Hinzu kommt noch, daß die Haarspitzen zuerst auf den Wickler gewickelt und dadurch stärker gekrümmt werden als der darüber liegende Haaransatz.

Ziel einer dauerhaften Haarverformung ist jedoch eine von den Haarspitzen bis zum Haaransatz gleichmäßige Verformung ohne Schädigung der Haarspitzen.

Eine Abschirmung der Wickler kann man durch die Anwendung von schaumförmigen Dauerverformungsmitteln erreichen. Dabei wird auf die Wickler eine geschlossene, stabile Schaumschicht aufgetragen. Nachteilig hierbei ist jedoch die nicht vermeidbare gleichzeitige Benetzung der Kopfhaut sowie das unkontrollierte Ablaufen der aus dem zerfallenden Schaum freigesetzten Verformungsflüssigkeit.

Es ist ferner bekannt, das Haar mit einem flüssigen Präparat, welches einen Gehalt von 0,35 Gew.-% Ammoniumsulfit bei pH = 7,2 aufweist, vorzufeuchten, zu wickeln und sodann ein dünnflüssiges Dauerverformunsmittel als Schaum auf das Haar aufzubringen. Während der Einwirkungszeit wird Wärme mittels eines Infrarotstrahlers zugeführt. Die geringe Mengen Ammoniumsulfit enthaltende Vorfeucht-Flüssigkeit ist jedoch nicht verformungswirksam, während das schaumförmige Dauerverformungsmittel wegen seiner Dünnflüssigkeit und der Kapillarwirkung des Haares rasch bis in die innersten Lagen des gewickelten Haares – also den Haarspitzen – eindringt. Eine kontinuierliche – vom Haaransatz zu den Haarspitzen hin – abnehmende Verformungswirkung wird daher nicht erreicht.

Aus der japanischen Friseurzeitschrift "New Hair Collection '78" der Men's Hairdressing Federation of Japan vom 01.10.1977 ist ein Verfahren zur Dauerwellung des Haares von männlichen Personen, welches auf eine Länge von etwa 7 cm vorgeschnitten wird, bekannt. Dort wird für die Haare im Bereich des Oberkopfes empfohlen, das Haar mit einer schwachen Dauerwellösung zu wickeln und auf das Haar im Bereich der Haarwurzel eine viskose, stark wirksame Dauerwellösung aufzutragen. Als Ergebnis sollen der Haaransatz stark und die Haarspitze sanft dauergewellt sein. Bei dieser Methode sind die Hände des Friseurs während der gesamten für das Wickeln benötigten Zeit von etwa 30 Minuten der Einwirkung der schwachen Dauerwellösung ausgesetzt.

Mit der in der deutschen Offenlegungsschrift 2 822 125 empfohlenen Abschirmung der Wickler mittels einer Schicht eines feinteiligen Feststoffes oder durch das Abdecken des Haares mit einer Kunststoffhaube wird zwar ein gewisser Erfolg hinsichtlich der Abschirmung erreicht, jedoch die gegenüber einer chemischen Umformung unterschiedliche Empfindlichkeit des Haares zwischen Haaransatz und Haarspitze nicht ausgeglichen.

Aufgabe der Erfindung ist daher ein Verfahren zur dauerhaften Verformung von Haaren, bei dem die vor-

3

stehend geschilderten Nachteile vermieden werden und auf einfache Weise eine zuverlässige und gleichmäßige Umformung des Haares erzielt wird.

Es wurde nun gefunden, daß die Aufgabe der Erfindung durch ein Verfahren zur dauerhaften Verformung von Haaren, bei dem man die gewickelten Haare mit einem konsistenten haarkeratinreduzierenden Dauerverformungsmittel mit einer Viskosität von 50 bis 5000 m Pa · s/bei 30°C, gemessen mit einem in der Kosmetikindustrie üblicherweise verwendeten Viskosimeter, behandelt, dadurch gekennzeichnet, daß man zuvor auf das Haar nach dem Aufrollen auf Wickler ein flüssiges haarkeratinreduzierendes Dauerverformungsmittel mit einer gegenüber dem konsistenten Dauerverformungsmittel schwächeren Verformungswirksamkeit aufträgt, nach einer ausreichenden Einwirkungszeit das Dauerverformungsmittel-Gemisch vom Haar abspült und das Haar mit einer oxidierend wirkenden Lösung behandelt, in hervorragender Weise gelöst ist.

Das bei dem erfindungsgemäßen Verfahren zu verwendende flüssige Dauerverformungsmittel sowie auch das konsistente Dauerverformungsmittel enthalten jeweils als verformungswirksamen haarkeratinreduzierenden Wirkstoff Sulfit oder bestimmte Mercaptoverbindungen, insbesondere Salze oder Derivate der Thioglykolsäure wie Ammoniumthioglykolat, Ammoniumthiolacetat und Glycerinmonothioglykolat. Es ist dabei jedoch nicht erforderlich, daß das flüssige und das konsistente Dauerverformungsmittel jeweils den gleichen haarkeratinreduzierenden Wirkstoff enthalten.

Als flüssige Dauerverformungsmittel kommen alle für das Haar schwach verformungswirksamen flüssigen Dauerverformungsmittel in Betracht, die bei den üblichen Einwirkungszeiten von etwa 10 bis 30 Minuten zwar in der Lage sind, das Haar im Bereich der Haarspitzen zu verformen, deren Verformungswuirksamkeit jedoch zu gering ist, um die Haarspitzen in diesem Zeitraum zu schädigen. Geeignete flüssige Dauerverformungsmittel sind zum Beispiel solche mit einem Gehalt von etwa 4 bis 10 Gew.-% Ammoniumthioglykolat und einem pH-Wert von etwa 7-8. Weitere geeignete flüssige Dauerverformungsmittel können den nachfolgenden Beispielen entnommen werden.

Das bei dem hier beschriebenen Verfahren verwendete konsistente Dauerverformungsmittel weist bei einer Temperatur von 30°C eine Viskosität von 50-5000 m Pa · s (Millipascalsekunden) auf und besitzt eine höhere Verformungswirksamkeit als das zuvor auf das Haar aufgebrachte flüssige Dauerverformungsmittel.

Da die Verformungswirksamkeit eines Dauerverformungsmittels nicht allein von der Konzentration des darin enthaltenen haarkeratinreduzierenden Wirkstoffs und der eingestellten Alkalität abhängt, sondern weitere enthaltene Stoffe, wie zum Beispiel bestimmte pflegende Zusätze, einen Einfluß auf die Verformungswirksamkeit haben, wird sie am sichersten experimentell vergleichend festgestellt. Verformungswirksamer ist dasjenige Dauerverformungsmittel, das unter gleichen Anwendungsbedingungen, das heißt, bei Verwendung gleicher Wickler, bei gleicher Temperatur und bei gleichen Zug beim Wickeln, am gleichen Haar die gleiche Verformung in kürzerer Zeit ermöglicht.

Das konsistente Dauerverformungsmittel kann zum Beispiel auf Grund folgender Faktoren verformunswirksamer sein als das flüssige Dauerverformungsmittel :

- Höhere Konzentration des haarkeratinreduzierenden Wirkstoffs
- Stärkere Wirksamkeit des haarkeratinreduzierenden Wirkstoffs
- Höherer Gehalt an Alkalisierungsmittel (höherer pH-Wert)

Selbstverständlich kann eine höhere Verformungswirksamkeit auch das Ergebnis einer Kombination der vorstehend genannten Faktoren sein.

Das konsistente Dauerverformungsmittel stellt im übrigen eine Dispersion, eine Emulsion oder ein Gemisch einer wäßrigen Lösung eines bekannten haarkeratinreduzierenden Wirkstoffs und gegebenenfalls eines Alkalisierungsmittels mit geeigneten Füllstoffen, Verdickern und/oder Emulgatoren sowie in Haarverformungsmitteln üblichen Zusätzen dar, das durch geeignete Wahl von Art und Menge der enthaltenen Füllstoffe, Verdicker und/oder Emulgatoren auf eine Viskosität von 50 bis 5000 m Pa · s, vorzugsweise 100 bis 500 m Pa · s, bei 30° C, eingestellt ist. Insbesondere liegt es in Form eines Gels oder einer Creme vor.

Als geeignete Füllstoffe kommen beispielsweise Kaolin, Montmorillonit und/oder Calciumcarbonat in Betracht, während als Verdicker zum Beispiel Weizenmehl, Tylose, Stärke, Gelatine, Cellulosederivate und Carboxyvinylpolymerisate dienen können. Als Verbindungen, die in Kombination mit einem geeigneten Emulgator stabile Cremes ergeben, sind beispielsweise höhere Fettalkohole, Fettsäureester, flüssige und feste Paraffine, Isoparaffine, Vaseline und Wollwachs zu nennen. Geeignete Emulgatoren sind unter anderem Fettalkoholsulfate, Fettsäurealkanolamide, Alkylsulfonate, Alkylbenzolsulfonate, oxethylierte Fettalkohole und oxyethylierte Nonylphenole. Außerdem können sowohl das flüssie als auch das konsistente Dauerverformungsmittel in kosmetischen Mitteln übliche Zusatzstoffe, wie zum Beispiel Antioxidantien, Parfümöle, Komplexbildner, Farbstoffe, niedere aliphatische Alkohole wie Ethanol und Isopropanol, Netzmittel, Trübungsmittel, schützende und pflegende Zusatzstoffe wie Harnstoff, Lanolinderivate, Cholesterin und Pantothensäure erhalten.

Bei dem erfindungsgemäßen Verfahren wird das gewaschene und handtuchtrocken gemachte Haar

4

zunächst in Partien aufgeteilt, auf Wickler gewickelt und dann erst mit dem flüssigen Dauerveformungsmittel durchtränkt. Um die Hände des Friseurs zu schonen, ist die Methode, erst die auf Wickler gewickelten Haare mit der flüssigen Dauerverformungsflüssigkeit zu durchfeuchten, vorzuziehen. Hierbei kommen die Hände des Friseurs nicht mit dem Dauerverformungsmittel in Kontakt. Auf das mit dem flüssigen Dauerverformungsmittel durchfeuchtete und auf Wickler gewickelte Haar wird nun das konsistente, stärker verformungswirksame Dauerverformungsmittel in gleichmäßiger geschlossener Schicht aufgetragen. Zum Auftragen kann das konsistente Dauerverformungsmittel direkt aus einer Tube oder einer Druckgaspackung gepreßt oder aber mit einem Hilfsmittel wie einem Pinsel oder Spatel auf das Haar gebracht werden.

Nach einer ausreichenden Einwirkungszeit, welche je nach Haarbeschaffenheit, Temperatur (die Anwendungstemperatur beträgt etwa 25 bis 50°C) und gewünschtem Grad der Umformung, bei etwa 10 bis 30 Minuten liegt, wird das gewickelte Haar gründlich mit Wasser gespült und dann durch Behandeln der Haare mit der wäßrigen Lösung eines Oxidationsmittels in der neuen Form fixiert.

Das neue erfindungsgemäße Verfahren zur Dauerverformung von Haaren weist gegenüber bekannten Verfahren zahlreiche Vorteile auf.

Es wirkt auf die empfindlichen Haarspitzen nur das flüssige, schwach verformungswirksame Dauerverformungsmittel ein, da das später aufgetragene konsistente, stärker verformungswirksame Dauerverformungsmittel nicht bis in die innersten Lagen des gewickelten Haares eindringen kann. Durch das konsistente Dauerverformungsmittel wird schließlich eine Abschirmung der Wickler erreicht. Diese Abschirmung verhindert weitgehend den Zutritt von Luftsauerstoff und verringert gleichzeit das Entweichen flüchtiger Alkalisierungsmittel wie Ammoniak sowie das Austrocknen der obersten Lage des gewickelten Haares erheblich.

Der abschirmende Effekt des konsistenten Dauerverformungsmittels läßt sich anhand folgender Experimente demonstrieren :

Zwei Haarsträhnen von gleichem Gewicht und gleichem Haar werden auf Wickler mit gleichem Durchmesser gewickelt und mit der gleichen Menge einer Dauerverformungsflüssigkeit befeuchtet. Anschließend wird auf einen der Wickler ein erfindungsgemäßes konsistentes Dauerverformungsmittel nach Beispiel 3 aufgetragen.

Verwendet man als Dauerverformungsflüssigkeit eine 8 Gew.-% Cystein als Reduktionsmittel enthaltende wäßrige Lösung, so beträgt der Verlust an Reduktionsmittel innerhalb einer Stunde ohne Abschirmung 45%, während mit Abschirmung lediglich ein Verlust von 7% auftritt.

Stülpt man über einen Wickler, der mit einem schwach sauer eingestellten Dauerverformungsmittel auf der Basis von Monothioglykolsäureglycerinester getränkt ist, eine Glocke und bringt ein Bleiacetatpapier unter die Glocke, so wird das Bleiacetatpapier durch den aus dem Wickler durch Zersetzung entweichenden Schwefelwasserstoff schwarz gefärbt. Wird der Wickler jedoch mit einem erfindungsgemäß zu verwendenden konsistenten Dauerverformungsmittel abgedeckt, so bleibt ein entsprechender Schwefelwasserstoffnachweis aus.

Schließlich wird durch die ausschließliche Einwirkung des stärker verformungswirksamen konsistenten Dauerverformungsmittels auf den Haaransatz auch in diesem kritischen Bereich der Haare eine erwünschte gute Umformung erreicht.

Das erfindungsgemäße Verfahren ist gleichermaßen gut für die Dauerwellung und Haarentkräuselung geeignet. Hierbei ist lediglich der Durchmesser der zu verwendenden Wickler, von 5 bis 13 Millimetern bei der Dauerwellung und über 13 Millimeter bei der Haarentkräuselung, der entsprechenden Anwendung anzupassen.

Die nachstehenden Beispiele sollen den Gegenstand der Erfindung näher erläutern.

Beispiel

Verfahren zur Dauerwellung von Haaren

Die gewaschenen, handtuchtrockenen Haare werden auf Dauerwellwickler gewickelt und anschließend mit der Gesamtmenge von 50 g eines flüssigen Dauerverformungsmittels A2 der Zusammensetzung

A2) Flüssiges Dauerverformungsmittel

4,0 g Monothioglykolsäureglycerinester
46,0 g Wasser
50,0 g

das einen pH-Wert von 6,5 und eine Viskosität vom 0,8 m Pa · s bei 30°C aufweist, gründlich befeuchtet.

5

Sodann wird mit einem Pinsel ein konsistentes und gegenüber A2 stärker verformungswirksames Dauerverformungsmittel B2 oder C2, der nachstehenden Zusammensetzung, auf das Haar aufgetragen.

B2) Konsistentes Dauerverformungsmittel

    9,5 g  Ammoniumthioglykolat, 50%ige wäßrige Lösung
    1,5 g  Ammoniumhydrogencarbonat
    1,5 g  Ammoniumcarbonat
    0,7 g  Ammoniak, 25%ige wäßrige Lösung
    0,3 g  1,4-Nonylphenol, mit 10 Mol Ethylenoxid oxethyliert
    1,3 g  Carboxyvinylpolymerisat (CARBOPOL 934® der Firma B. F. Goodrich, Cleveland/Ohio, USA)
    0,2 g  Parfümöl
    <u>35,0 g</u>  Wasser
    50,0 g

(Dieses Dauerverformungsmittel in Gelform hat einen pH-Wert von 8,8 und eine Viskosität von 3600 m Pa · s bei 30° C)

C2) Konsistentes Dauerverformungsmittel

    16,0 g  wäßrige, 33%ige Ammoniumsulfitlösung
    10,0 g  Imidazolidinon-2
    0,8 g  Tylose
    2,8 g  Isopropanol
    0,2 g  Parfümöl
    <u>20,2 g</u>  Wasser
    50,0 g

(Dieses Dauerverformungsmittel in Gelform hat einen pH-Wert von 6,5 und eine Viskosität von 1900 m Pa · s bei 30° C)

Man bedeckt, das Haar mit einer Plastikhaube und führt mit Hilfe einer Trockenhaube Wärme zu. Nach einer Einwirkungszeit von 10 Minuten unter der Trockenhaube wird die Plastikhaube entfernt, das Haar gründlich mit lauwarmem Wasser gespült und mit einer Papierserviette von überschüssigem Wasser befreit. Schließlich wird das Haar zur Fixierung mit einer 10%igen wäßrigen Lösung von Natriumbromat behandelt.

## Ansprüche

1. Verfahren zur dauerhaften Verformung von Haaren, bei dem man die gewickelten Haare mit einem konsistenten haarkeratinreduzierenden Dauerverformungsmittel mit einer Viskosität von 50 bis 5000 m Pa · s/bei 30° C, gemessen mit einem in der Kosmetikindustrie üblicherweise verwendeten Viskosimeter, behandelt, dadurch gekennzeichnet, daß man zuvor auf das Haar nach dem Aufrollen auf Wickler ein flüssiges haarkeratinreduzierendes Dauerverformungswirksamkeit aufträgt, nach einer ausreichenden Einwirkungszeit das Dauerverformungsmittel-Gemisch vom Haar abspült und das Haar mit einer oxidierend wirkenden Lösung behandelt.

2. Verfahren nach Anspruch 1, dadurch gekennzeichnet, daß das flüssige und das konsistente Dauerverformungsmittel als haarkeratinreduzierenden Wirkstoff jeweils eine Mercaptoverbindung enthalten.

3. Verfahren nach Anspruch 1 und 2, dadurch gekennzeichnet, daß die Mercaptoverbindung ein Salz oder ein Ester der Thioglykolsäure ist.

4. Verfahren nach Anspruch 1 bis 3, dadurch gekennzeichnet, daß das konsistente haarkeratinreduzierende Dauerverformungsmittel bei 30°C eine Viskosität von 100 bis 500 m Pa · s aufweist.

5. Verfahren nach Anspruch 1 bis 4, dadurch gekennzeichnet, daß das konsistente Dauerverformungsmittel in Form eines Gels oder einer Creme vorliegt.

6. Verfahren nach Anspruch 1 bis 5, dadurch gekennzeichnet, daß die Einwirkungszeit des konsistenten Dauerverformungsmittels auf das Haar etwa 10 bis 30 Minuten bei einer Temperatur von 25 bis 50°C beträgt.

7. Verfahren nach Anspruch 1 bis 6, dadurch gekennzeichnet, daß das flüssige Dauerverformungsmittel eine schwache Verformungswirksamkeit aufweist, welche zwar ausreicht, das Haar im Bereich der Haarspitzen zu verformen, jedoch nicht ausreicht, bei der Einwirkungszeit von 10 bis 30 Minuten die Haarspitzen zu schädigen.

8. Verfahren nach Anspruch 1 bis 7, dadurch gekennzeichnet, daß das flüssige Dauerverformungsmittel etwa 4 bis 10 Gew.-% Ammoniumthioglykolat enthält und einen pH-Wert von etwa 7 bis 8 aufweist.

9. Verfahren nach Anspruch 1 bis 8, dadurch gekennzeichnet, daß das Verfahren ein Verfahren zur Dauerwellung von Haaren ist.

## Claims

1. Process for the permanent shaping of hair, in which the rolled hair is treated with a viscous hair keratin reducing permanent shaping medium having a viscosity from 50 to 5000 m Pa · s at 30° C measured with a viscosity meter normally used in the cosmetics industry, characterised in that there is previously applied to the hair, after rolling onto curlers, a fluid hair keratin reducing permanent shaping medium with weaker shaping activity than the viscous permanent shaping medium, the permanent shaping medium mixture is rinsed out of the hair after acting for a sufficient time, and the hair is treated with an oxidatively-acting solution.

2. Process according to claim 1, characterised in that the fluid and viscous permanent shaping medium each contain a mercapto compound as hair keratin reducing agent.

3. Process according to claim 1 and 2, characterised in that the mercapto compound is a salt or an ester of thiglycolic acid.

4. Process according to claim 1 to 3, characterised in that the viscous hair keratin reducing permanent shaping medium has a viscosity from 100 to 500 m Pa · s at 30°C.

5. Process according to claims 1 to 4, characterised in that the viscous permanent shaping medium is in the form of a gel or cream.

6. Process according to claims 1 to 5, characterised in that the time of action of the viscous permanent shaping medium on the hair amounts to about 10 to 30 minutes at a temperature of from 25 to 50°C.

7. Process according to claims 1 to 6, characterised in that the fluid permanent shaping medium has a weak shaping activity which is nevertheless sufficient to shape the hair in the region of the hair ends, but is however not sufficient, over a time of action of from 10 to 30 minutes, to damage the hair ends.

8. Process according to claims 1 to 7, characterised in that the fluid permanent shaping medium contains about 4 to 10 weight per cent of amonium thioglycolate and has a pH value of about 7 to 8.

9. Process according to claims 1 to 8, characterised in that the process is a permanent waving process for hair.

## Revendications

1. Procédé de déformation durable des cheveux dans lequel on traite les cheveux enroulés avec un agent consistant de déformation durable agissant comme réducteur de la kératine des cheveux, ayant une viscosité de 50 à 5000 m Pa · s à 30°C (millipascals × seconde) mesurée avec un viscosimètre utilisé habituellement dans l'industrie cosmétique, caractérisé par le fait que :

on applique auparavant à la chevelure, après l'enroulement sur des rouleaux, un agent liquide de déformation durable agissant comme réducteur de la kératine des cheveux et ayant un pouvoir déformant plus faible que celui de l'agent consistant de déformation durable,

après un temps suffisant d'action on rince la chevelure pour éliminer le mélange d'agents de déformation durable et on traite la chevelure avec une solution ayant une action oxydante.

2. Procédé selon la revendication 1, caractérisé en ce que l'agent liquide et l'agent consistant de déformation durable contiennent l'un et l'autre comme réducteurs de la kératine des cheveux un composé mercapto.

3. Procédé selon les revendications 1 et 2, caractérisé en ce que le composé mercapto est un sel ou un ester de l'acide thioglycolique.

4. Procédé selon les revendications 1 à 3, caractérisé en ce que l'agent consistant de déformation durable agissant comme réducteur de la kératine des cheveux présente à 30°C une viscosité de 100 à 500 m Pa · s (millipascals × seconde).

5. Procédé selon les revendications 1 à 4, caractérisé en ce que l'agent consistant de déformation durable est sous la forme d'un gel ou d'une crème.

6. Procédé selon les revendications 1 à 5, caractérisé en ce que le temps d'action de l'agent consistant de déformation durable agissant sur la chevelure est approximativement de 10 à 30 minutes à une température de 25 à 50°C.

7. Procédé selon les revendications 1 à 6, caractérisé en ce que l'agent liquide de déformation durable présente un pouvoir déformant faible, bien que suffisant pour déformer les cheveux dans leur zone terminale, tout en étant suffisamment réduit pour ne pas endommager les pointes des cheveux lorsqu'il agit pendant une durée de 10 à 30 minutes.

8. Procédé selon les revendications 1 à 7, caractérisé en ce que l'agent liquide de déformation durable contient approximativement de 4 à 10% en poids de thioglycolate d'ammonium et présente un pH d'environ 7 à 8.

9. Procédé selon les revendications 1 à 8, caractérisé en ce que le procédé est un procédé d'ondulation permanente des cheveux.